# EUROPEAN PATENT SPECIFICATION

(11) **EP 1 176 970 B1**
(45) Date of publication and mention of the grant of the patent: **19.05.2010**
(21) Application number: 00925757.7
(22) Date of filing: 28.04.2000
(51) Int. Cl.: A61K 35/30, A61L 27/38, A61P 25/00

(54) **XENOTRANSPLANT FOR CNS THERAPY**
XENOTRANSPLANT FÜR ZNS THERAPIE
XENOTRANSPLANT POUR LE TRAITEMENT DU SYSTEME NERVEUX CENTRAL

(30) Priority: 30.04.1999 NZ 33555399
(43) Date of publication of application: 06.02.2002
(73) Proprietor: NeurotrophinCell Pty Ltd, Sydney NSW 2001 (AU)
(72) Inventor: ELLIOTT, Robert, Francis, Remuera, Auckland 1130 (NZ); SKINNER, Stephen, John, Martin, Lower Hutt 6304 (NZ); WILLIAMS, Christopher, Edward, Melbourne Victoria (AU)
(74) Representative: Wright, Simon Mark
(86) International application number: PCT/NZ2000/000064
(87) International publication number: WO 2000/066188

(56) References cited:
- US-A- 5 389 535
- US-A- 5 550 050
- US-A- 5 869 463
- US-A- 5 871 767
- KITADA MASAAKI ET AL: "Axonal regeneration in the central nervous system is enhanced by ependymal cell transplants." NEUROSCIENCE RESEARCH SUPPLEMENT, no. 22, 1998, page S318 XP000960785 21st Annual Meeting of the Japan Neuroscience Society and the First Joint Meeting of the Japan Neuroscience Society and the Japanese Society for Neurochemistry;Tokyo, Japan; September 21-23, 1998 ISSN: 0921-8696

## Description

### FIELD

This invention relates to a composition for use in the treatment of some neurological diseases of the central nervous system of a mammal and more particularly to compositions including living cells derived from a mammal, and in particular to a composition and method of use employing the living cells to secrete factors, over a period, capable of having a desired effect on the central nervous system.

### BACKGROUND

Significant neurodegenerative diseases of the central nervous system (CNS) include Alzheimer's disease (AZ), multiple sclerosis (MS) and Parkinson's disease (PD). In the United States and Europe alone, the incidence of AZ is estimated at 8 million; MS at 0.7 million, and PD at 1.5 million. There are of course many other diseases; epilepsy, Huntington's chorea, stroke, and so on. At this time all available treatments would appear to be palliative rather than restorative and the inevitable progress of these diseases is slowed, perhaps, but not reversed.

The assumption that neurones cannot regenerate has constrained past approaches for treatments of diseases of the central nervous system. Furthermore, therapy of the central nervous system (CNS) is more difficult than for the remainder of the body in part because of the "blood-brain barrier" which is a concept used to describe a functional obstacle to the entry of some materials including therapeutic materials from the systemic circulation. The barrier resides, in functional terms, around all (normal) capillary structures within the CNS. Morphologically, altered pinocytotic behaviour and tight junctions of the endothelial cells are characteristic. Introduction of foreign substances directly into the CNS, such as into the ventricles is a good deal more difficult, unpleasant, and dangerous than taking a pill four times daily. In addition, a particular and quite separate version of "lymph circulation" within the CNS - the circulation of cerebrospinal fluid - tends to remove any material that does cross the barrier. Lymphatics themselves do not extend to the CNS.

There is also a knowledge barrier. For example a widely held belief is that the cerebrospinal fluid does not perfuse the substance of the brain in a manner capable of carrying materials about, while a few, including ourselves, believe that it does provide an effective perfusion medium capable of distributing trophic factors about most, if not all of the parenchyma of the CNS making use of white matter tracts, perivascular spaces and the like. There has been experimental evidence for that widely held belief, e.g. Brightman & Reese, or Blasberg Patlack & Fenstermacher, (cited in W M Pardridge "Transnasal and intraventricular delivery of drugs" in "Peptide drug delivery to the brain" ed. W M Pardridge, New York: Raven Press 1991) involving limited distances achieved by the intraventricular infusion of a selection of traceable compounds. Most prior art known to us appear to be based on local diffusion, such as US 5853385, or US 5573528 "Implanting devices for the focal release of neuroinhibitory compounds" to Aebischer & Tresco. Krewson et al (Brain Res 1995 May 22 680 (1-2) 196-206 state that nerve growth factor travelled only 2-3 mm from a polymer insert through rat brain tissue. This paper also exemplifies the "single-factor" approach. See later. A knowledge gap also extends in relation to the interplay between trophic substances (such as insulin growth factors including IGF-II and the like, also nerve growth factor or NGF,) and their normal regulation and site or sites of production at different stages of life including the fetus. Walter HJ et al (Endocrinology 140(1) 520-32) considers that IGF-II secretion from the choroid plexus of an injured rat brain is raised as a response to injury, "resulting in an increased transport of the peptide to the wound".

An increasing number of conditions of the central nervous system capable of responding to therapy are being recognised. It is interesting to note that cerebrospinal fluid production is impaired in a number of such conditions and furthermore it is possible that there is a loss of paracrine factors such as growth factors, in the case of specific diseases.

In many cases the indicated therapeutic agent for a restoration therapy or the like is a naturally occurring cell secretion, for example a polypeptide (such as IGFII) rather than an exogenous substance such as an antibiotic derived from a fungus or bacterium. Substantially continuous application throughout the entire CNS over a long period is acceptable in most of these treatments.

Within the patent literature, Patrick Aebiscber and associates have filed many patents dealing with implants, including both live cells and manufactured slow-release formulations into specified parts of the CNS; for example US5389535 for manufacturing a tubular cell-carrying implant. WO99/56770 to Chang is possibly most similar to the present application, in that Chang teaches the injection of microcapsules holding specified live cells, capable of releasing an enzyme lacking in a lysosomal storage disease, into a ventricle. Cells known as "neural stem cells" are used by Carpenter in US 5968829 to CytoTherapeutics Inc; such cells are undifferentiated cells capable of evolving into either neurones or glial cells. A commercial application was absent. Many documents (e.g. US 5898066 for trophic factors (axogenesis factors), WO99/36565 (human ependymin), US 5573528 (neuroinhibitory compounds such as GABA for control of involuntary movement)) deal with specific substances. A number of documents disclose the implantation of live cells. For example, Gage et al (US 5762926) exemplifies genetically modified live-cell grafts, and Holland et al (US 5550050) describes exposure of live cells to restrictive conditions prior to implantation; both so that the resulting implant functions in the intended manner. In addition, Major et al (US 5869463) describes the use of human foetal neuro-derived cells which are transformed by genetic modification into a non-inflammatory immortalised cell line. Major *et al* further describe the encapsulation of such cells along with genetic modification to present immune response upon implantation. The use of encapsulation to further protect the cells from an immune response is also disclosed by Major *et al* containing a nucleic acid sequence encoding a biologically active peptide. Whilst Kitada Masaaki et al (Neuroscience Research Supplement, No. 22, 1998, 21st Annual Meeting of the Japan Neuroscience Soc. and the First Joint Meeting of the Japan Neuroscience Society and the Japanese Society for Neurochemistry; Tokyo, Japan; Sept 21-23, 1998) have examined the effect of ependymal cell transplants in facilitating axonal regeneration, there is little published.

There is little published material dealing with "factors leading to rejuvenation" and no patents take advantage of the differentiated, very active cells of the choroids plexus.

The problem to be solved is to identify an effective treatment for at least one neurological disease.

### DEFINITIONS

A **"neurological disease"** covers any disorder of the central nervous system. It may for example be a global neurodegenerative disease, such as ageing, vascular disease, Alzheimer's disease, or the more localised Parkingson's disease, or the autoimmune disease multiple sclerosis (MS); it may be a result of injury, such as a stroke, anoxia/asphyxia, or physical injury such as from a blow to the head; it may be a result of exposure to local (e.g.meningitis) or systemic toxins, and it may be neoplastic. It may be genetically based, such as Huntingdon's chorea, or a disorder of metabolism such as lysosomal storage disease

There is a group of "global neurodegenerative diseases" including AZ and others, affecting the elderly, the usual pattern of response to acute injury (such as ischaemia), affecting any age group including stroke victims and car accident victims, autoimmune diseases such as MS, PD, and certain diseases, including deficiencies of metabolism, of neonates and foetuses. Indeed PD may be more global than is currently appreciated. The known defects in and around the basal ganglia may be reflected elsewhere.

By **"restorative effect"** we include any beneficial modification of the disease process, including palliative, restorative, or proliferative effects acting on neural tissue, glia, or vascular elements. We tend to use "trophic" and "growth" factors interchangeably.

By **"rejuvenation"** we mean attempts to reverse changes in a brain commonly considered to be the usual, if not the normal consequences of ageing, such as loss of volume, loss or atrophy of neurones, loss of memory and loss of ability to cope with complex sensory inputs. Rejuvanation could also comprise restorative effects on existing neurones, neural rescue as required after an ashyxic episode, or "sick neurones".

### OBJECT

It is an object of this invention to provide apparatus and/or material and/or means for CNS therapy based on xenotransplantation of choroid plexus epithelium, or at least to provide the public with a useful choice.

### STATEMENT OF INVENTION

In a first broad aspect this invention provides a pharmaceutical composition, comprising an implantable device, for implantation into the brain of a recipient mammal suffering from a neurological disease, wherein the implantable device comprises living cells collected from the choroid plexus of a mammal wherein said cells are not collected from a human embryo, at least some of which cells are choroid epithelial cells, said implant secreting at least one product, having a beneficial effect on the neurological disease, into the brain of the recipient mammal characterised in that the living cells are placed within a biocompatible containment means, wherein the living cells are encapsulated within a biocompatible capsule, the wall of which is at least partially composed of a semi-permeable membrane adapted to admit metabolites for sustaining the cells while blocking access by factors of the immune system of the recipient mammal, said membrane also being adapted to allow an effective amount of one or more secreted products to exit from the implantable device.

Preferably all the living cells are derived from epithelial cells of the choroid plexus; alternatively some of the cells may be derived from other tissues of the choroid plexus or from other sources.

Preferably the pharmaceutical composition is adapted to survive its introduction within the brain of the mammal while producing the therapeutic agent, so as to permit treatment over, an extended period.

Preferably the membrane is also adapted to permit ingress of any substances capable of controlling the rate of release of the therapeutic agent.

Preferably the biocompatible capsule has an inner layer including an effective amount of a laminin; said laminin serving as a physical substrate for the living cells thereby providing orientation and support for the cells.

Preferably the biocompatible capsule comprises a globular containment means holding living cells.

More preferably the biocompatible capsule comprises a tubular containment means holding living cells; the implantable device being adapted to be placed within a ventricle of the brain of the recipient mammal, whereby the products secreted from the implantable device may access at least some regions of the central nervous system.

One preferred physical substrate is shaped like a hollow dialysis tube which holds living tissue (as previously described in this section) within a space within the CNS.

Another possible physical substrate comprises a closed mesh structure which retains cell groups inside biocompatible capsules within the closed structure so that the entire structure is removable as a unit.

Preferably at least one living epithelial cell is taken from the choroid plexus of a foetal or neonatal mammal having a selected age, so that at least some living cells have a predictable capability for secreting at least one product having a beneficial effect on a neurological disease.

Preferably the donor mammal is a non-human mammal.

Conveniently the donor mammal or at least the living material is free of infectious agents and preferably the donor mammal is from a stock kept under germ-free conditions.

Optionally the at least one living cell has undergone subsequent modification in order to increase the production of at least one product having a beneficial effect on a neurological disease.

Optionally the living material may comprise cultured cells; that is, separated by one or more generations from an initial isolate.

Preferably, treatments such as bFGF may be used to selectively enhance growth in culture.

Therapeutic agents include but are not limited to the naturally occurring peptides IGF-II, VEGF, TGF-alpha, NT-3 and bFGF.

Alternatively the living material comprises cells having a modified complement of genetic material capable of either secreting novel peptides

Alternatively the factors secreted may include naturally occurring peptides (such as one or more of those previously listed in this section), in altered amounts.

Alternatively, the peptides secreted may include compounds normally secreted elsewhere, such as thyroxine, insulin, or analogues thereof.

Alternatively the peptides secreted include sets of peptides secreted during different stages of development, such as peptides characteristic of foetal or neonatal choroid plexus cells, or analogues thereof.

Preferably the living material is also capable of being controlled by one or more endogenous control agents, or by one or more exogenous control agents.

Preferably the invention provides a container for transport and distribution, capable of holding at least one implant as previously described in this section, wherein the container also holds a liquid medium capable of maintaining the at least one implant in a living condition for a time during transport and storage.

Optionally the living material is provided in a state of suspended animation, suitable for storage and transport. Preferably this state is a cryopreserved state, although other forms of providing for the continuation of cell metabolism are included.

In a second broad aspect this invention provides a kit of materials for surgical implantation of an implantable device, as previously described in this section, in the central nervous system of a recipient mammal, the kit of materials including means for providing a sterile site, means for obtaining surgical access through the cranium to the central nervous system, means for haemostasis, means for placing at least one implantable device in an intended position, a container holding implantable devices as previously described in this section, means for closing off the surgical access site, and means for dressing the surgical access site, thereby to minimise the risk of introducing an infection during the operative procedure.

Optionally the kit of materials is restricted to means for placing at least one implantable device in an intended position and a container holding implantable devices as previously described in this section.

In a third broad aspect this invention provides a vascularised device or artificial choroid plexus for use with the body of a mammal to be treated, *wherein* the vascularised device includes (a) means to connect a first, blood-bearing compartment of the device between an artery and a vein, (b) means to pass a fluid carrying means leading from a second, transudate-bearing compartment of the device to an implantable second end of the fluid carrying means, adapted to be implanted into a space within the brain containing cerebrospinal fluid, and (c) internal support means, comprising a permeable wall between the first compartment and the second compartment, said wall being adapted to support at least one living cell collected from the choroid plexus of a mammal as previously described in this section, so that said living cells are exposed to transudate passing from the first compartment to the second compartment and so that said living cells express trophic factors into the transudate carried into the brain.

### PREFERRED EMBODIMENT

A preferred embodiment of the invention will now be described by way of non-limitative example with reference to the accompanying drawings.

### DRAWINGS

- Fig. 1:: Diagram of an encapsulated choroid plexus cell preparation. (Example 1)
- **Fig 2:**: Graph showing uptake of dopamine by cells exposed to media previously surrounding a choroid plexus cell preparation.
- **Fig 3:**: Photomicrograph of an encapsulated choroid plexus cell preparation
- **Fig 4:**: Photomicrograph of an encapsulated choroid plexus cell preparation
- **Fig 5:**: Diagram of an example dialysis tube implant for a choroid plexus cell preparation.

In summary, the invention (as embodied within this example) particularly comprises the use of living choroid plexus secretory cells used as an xenobiotic transplant or "artificial choroid plexus" placed most conveniently though not exclusively within the cerebral ventricles . The choroid plexus, situated within each lateral ventricle and also in the roof of the fourth ventricle is described as a highly vascularised substrate covered by epithelial cells which are in contact with the cerebrospinal fluid. A large number of villous processes provide an estimated surface area not including consideration of the apical microvilli of the epithelial (sometimes called ependymal) cells of over 200 square cm (adult human).

The choroid plexus is well-innervated vascular tissue (more correctly an organ) coveted With a basement membrane comprising the usual variants of collagen, one or more types of laminin, proteoglycans and other extracellular matrix molecules, which is in turn covered by an unicellular epithelium-like layer and occurring in several consistent sites within the cerebral ventricles. It appears to act as the source of most of the cerebrospinal fluid. Electron microscopy shows that the epithelial cells include a number of specialisations for protein synthesis and export including a dense layer of microvilli adjacent to the ventricle and adjacent to rough endoplasmic reticulum with ribosomes, yet relatively little Golgi apparatus, consistent with polypeptide secretion. Mitochondria are frequent Underlying the epithelium there are fenestrated endothelial cells of an almost continuous layer of capillaries.

The account of which we approve concerning the circulation of cerebrospinal fluid is as follows: The choroid plexus comprises a well-folded sheet of epithelial cells supplied with an extensive capillary bed, together with "a well-developed adrenergic and cholinergic nerve supply" (Lindvall M et al, Acta Physiologica Scand Suppl 1977; 452; 77-86). Most CSF originates within the choroid plexus tissue as combined ultrafiltrate and secretion, while a small amount originates in subarachnoid and perivascular spaces. This circulates unidirectionally through the cerebral aqueduct or aqueduct of Sylvius into the fourth ventricle, then through the median foramen of Magendie or lateral apertures of Luschka, then into and around the brain and spinal cord. The fate of most CSF is reabsorbtion into the blood at the arachnoid villi and through capillary walls. In the adult human about 430-450 ml of CSF is produced daily. Given that about 125-150 ml of fluid is present at any one time it follows that this amount is turned over every 6 or 7 hours. This unidirectional flow model does not include a clear path for putative substances from within neural tissue to reach and have any autoregulatory effect on the choroid plexus lying substantially at the "headwaters"; perhaps these travel via the blood or perhaps there is some reusage of CSF. Carriage of CSF through the parenchyma of the brain includes perivascular spaces, white matter tracts, and the like. Recently, Segal MB reviewed the choroid plexus (in Cell Mol Neurobiol 2000 Apr; 20(2) 183-196) and stated that "the CSF may act as a third circulation conveying substances secreted into the CSF rapidly to many brain regions". Note the term "rapidly".

Recent research suggests that the choroid plexus is likely to produce a number of trophic factors that co-ordinate cerebral development and thus anabolic processes. For example Zheng et al note that the thyroxin transport protein "transthyretin" (TTR) occurs in choroidal epithelial cells (and may serve as a diagnostic or assay feature to indicate activity of such cells). Age dependence of the trophic factors being produced is quite likely. Our previous research on xenobiotic transplants of pig pancreatic islet cells as an "artificial endocrine pancreas" provided a source of systemically available insulin which is responsive to autoregulation and the present invention provides an analogous approach to treatment of tissues usually regarded as behind the blood-brain barrier and therefore difficult to reach for treatment.

Indeed, Alzheimer's disease is sometimes called "diabetes of the brain". Whether or not this particular description is accurate, we expect to identity a number of syndromes where an artificial choroid plexus provides a useful form of treatment particularly in that it avoids repeatedly invading the CSF for the purposes of treatment. Myelinisation of axons in the central nervous system, such as during early postnatal life, presumably depends at least in part on a trophic factor.

It would appear that placement of an artificial choroid plexus comprising active epithelial cells behind a mutually protective barrier within a ventricle would provide a route for the introduction of substances into the CSF without having to cross a blood-brain barrier. Initially we have explored those substances naturally produced by choroid plexus epithelial cells. Known factors include: insulin-like growth factor (IGF-II), transforming growth factor alpha (TGF-a), retinoic acid (RA) which may be an essential trigger for neural differentiation, perhaps nerve growth factors (NGF), and possibly, because these factors are present in the CSF, vaso-endothelial growth factor (VEGF), and fibroblast growth factor (FGF). The choroid plexus also synthesises a variety of binding proteins which act as directed carriers of trophic factors.

### EXAMPLE 1

This example relates to the preparation of choroid plexus secretory cells suitable for encapsulation, and tests. All procedures are carried out in "GMP" licensed facilities, including strict infection barriers.

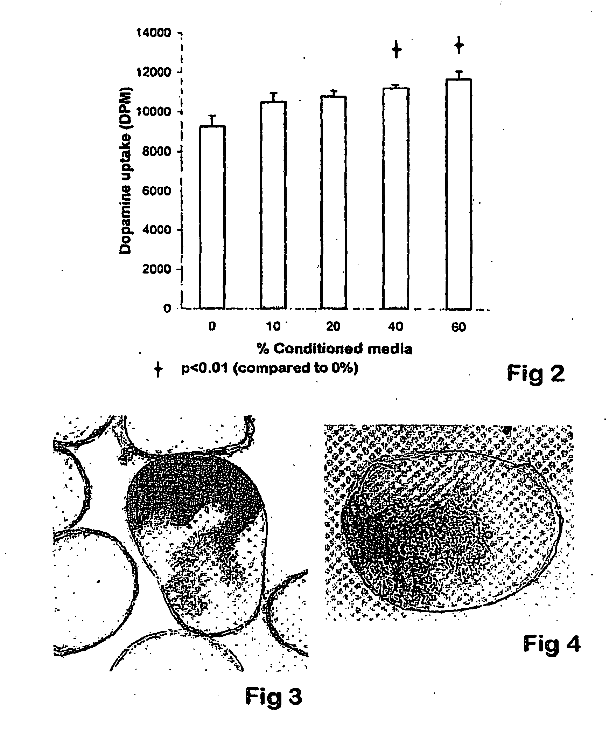

Late fetal or neonatal pigs are anaesthetised and their brains removed under aseptic surgical conditions, and cut in half to expose the plexus tissue. (We happen to have an SPF colony of pigs; but the invention is not limited to use of pig material).
(1) The cells producing the various soluble factors required are first freed from the surrounding tissues by collagenase digestion. The choroid plexus is removed from the piglet brain after as short a time of warm ischemia as possible. The plexus is minced and subjected to the digestion process in a suitable warm buffer with suitable mild agitation.
(2) The intact choroidal secretory cells are then separated from cellular debris and undigested tissue aggregates by filtration and then repeated centrifugation and washing in HBSS. They are then suspended in RPMI 1640 cell culture media, with added 2% human serum albumin and 1% penicillin/streptomycin.
(3) The cells are then inoculated into 6-well plates which may have received treatment to assist in the attachment of the cells to the inner surface. A preferred treatment comprises previous exposure of the well to a 0.025 mg/ml solution of laminin (Sigma L2020) for 2 minutes. (This is notably more successful in terms of surviving cells, which have spread about, than agarose).
(4) After a predetermined period of culture, typically 24 days at 37C, the cells are harvested and for example may be checked for viability by dye exclusion.
(5) The secretory functionality is checked using an *in vitro* culture, using the "conditioned media" removed from the choroid plexus cells. (see Fig 2 and Table 1) Further tests may include functional tests such as measures of the ability to produce IGF-II or TTF.

Note that this example procedure does not include any particular step to effectively exclude cells other than epithelial cells; there is a possibility that fibroblasts, endothelial cells, and the like co-exist, may assume similar *in vitro* growth patterns, and may interact. There is a possibility that useful inter-relationships between choroid epithelial cells and other cells not from the choroid plexus may be exploited in order to make a more effective implant.

**Assessment of neurotrophic bioassay technique for testing conditioned media from processed choroid cells.**

For this experiment cells of the Sks cell line (a neuroblastoma) were plated into a sterile 96-well plate at 10,000 cells per well and after 24 hours "settling" at 37 deg C in humidified air, the original media was replaced by varying proportions of conditioned media as described above. Then the cells were visually assessed and scored according to the amount of growth, the number of dendrites, and connections overlapping other cells. The 50% conditioned media group grew dense connections and dendrites which covered the whole well.

**Table 1: Neuronal growth rate for a Sks cell line, scored after 36 hours exposure to conditioned media.**

| | ***100% CM*** | ***50% CM*** | ***10% CM*** | ***1% CM*** | ***control*** |
|---|---|---|---|---|---|
| *Row 1* | +++ | ++++ | ++ | + | + |
| *Row 2* | +++ | ++++ | ++ | + | + |
| *Row 3* | ++++ | +++++ | ++ | + | + |
| *Row 4* | +++ | ++++ | ++ | + | + |

### A dopamine uptake bioassay technique for testing conditioned media from processed choroid cells (see Fig 2).

For this experiment, mesencephalon (Mes) cells - which would include glia and neurones - were isolated from E15 rat fetuses and incubated in MEM plus 5% fetal bovine serum. Next day the cell media was aspirated, the wells were rinsed once with MEM, 400 microlitres of N2 media added (including selenium, progesterone. BSA, insulin and transferrin) and 600 microlitres of RPMI media was added, containing conditioned media, to a final concentration of 0, 20%. 40% and 60%. The RPMI includes 10 mM nicotinamide and 5% human serum albumen. The cells were incubated for 36 hours and then standard dopamine and GABA uptake assays were performed. The results are illustrated in Fig 2, showing that dopamine uptake rises as the concentration of conditioned media is increased.

### Encapsulation of the choroid secretory cells

All procedures are carried out in GMP-certified aseptic conditions. Clumped choroid cells are suspended in a suitable sterile solution of alginate. Preferably the alginate is assured to contain minimal lipopolysaccharide and endotoxins. Preferably the alginate includes a substrate material such as a laminin, or the like, in order to assist cell settling. The alginate coating of the suspended cell clumps is then hardened and rendered insoluble by exposure to calcium ions after suitable dispersion. The electrical charge on the alginate coating is then neutralised using polylysine or polyomithine. The encapsulated material is then further coated with alginate and hardened.

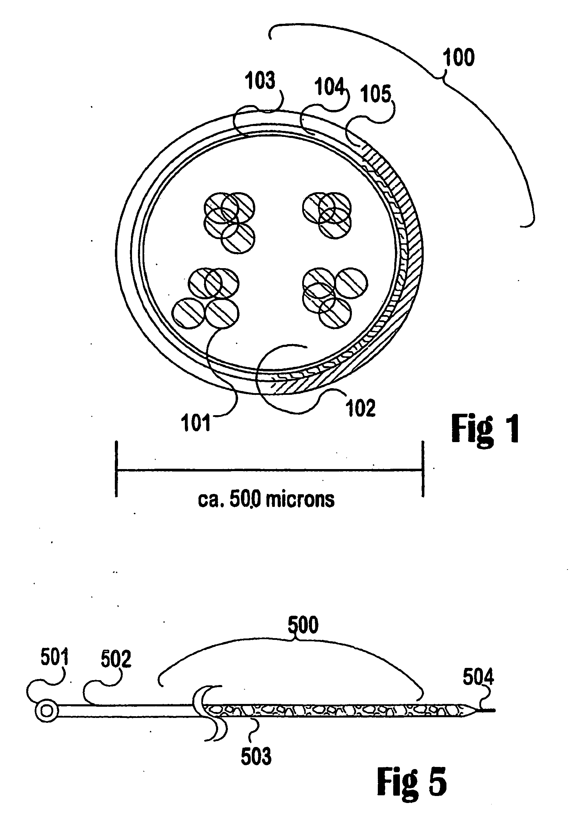

Figure 1 shows an example cell / coating structure 100. Here, clumps of cells 101 float in liquid 102 within a hollow sphere made up of several layers or coatings 103, 104, 105 which may be alternating alginate, polyornithine, and alginate layers. Preferably the innermost layer at least includes laminin. Figures 3 and 4 are photomicrographs showing actual globular containers built with alginate and containing choroid epithelial cells. The encapsulated cell clumps are then cultured further in physiological conditions with the addition of 10mM nicotinamide. Thus a suspension of cell clumps each around 50 microns in diameter is made. In many ways, a pharmaceutical composition which is a simple suspension of this type may be the most convenient mode of delivery. Perhaps delivery can be by infusion through a spinal tap; relatively easy to perform.

We propose that an artificial choroid plexus according to this example is capable of secreting trophic substances including the known items listed previously, plus uncharacterised trophic factors such as analogues of IGF-II or a variety of peptides, thereby having a beneficial influence on the cells of the central nervous system by a paracrine mechanism. Some of the effective trophic substances have not as yet been identified. This is inherent in the use of extracts from a naturally occurring organ rather than an engineered cell line. Note that endogenous growth factor binding proteins may play a useful part in this invention for carrying the growth factors from the ventricle to the site of action within the parenchyma of the CNS; examples being IGF-BP2 and possibly follistatin, GH-BP.

### EXAMPLE 2

Thread-like single implants are for several reasons preferred over a loose suspension of globules containing cells. For example, if a single globule breaks, the cells within may be released with adverse consequences such as of immunological rejection, or transfer of latent virus infection that may be carried by the cells. Also, neurosurgeons are understood to prefer to use threads because they resemble existing tubular implants such as shunts, and drainage and monitoring catheters for use in the intracranial ventricles. Surgical techniques for the placement and later removal of these are well established. An ability to remove implants according to the invention is likely to be useful. A significant amount of medical technology exists (e.g. "Medtronic", California) in relation to shunts, and drainage and monitoring catheters for use in the intracranial ventricles. Therefore, example 2 comprises compatible objects for the delivery of xenotransplants in the form of living cells within selectively permeable tubing. Such tubing comprises a kind of disposable, implantable device that carries either an inner surface lining of a laminin in order to induce the cells to settle, or includes a cavity holding protected choroid plexus cells as described above, which can be left in place for a long period of perhaps months.

All these systems involve the usual precautions (sterility and care) needed for an intracranial operation; however it may be possible to perform it under a local instead of a general anaesthetic, and hence the procedure is more compatible with use in developing countries, and/or where costs should be minimised.

Surgical techniques for the implantation of a composition according to the invention can be carried out, preferably into a lateral ventricle, and preferably from a frontal, parietal, or occipital approach. The occipital approach, being more or less in line with the long axis of a lateral ventricle, allows a longer "artificial choroid" to be deposited. (It should be noted that in many of the conditions considered as appropriate for this type of treatment, stereotaxic techniques are difficult if not impossible owing to the brain becoming distorted). An example artificial choroid may comprise one or a bundle of dialysis-type hollow fibres containing free cells. Optionally, the fibre may be a tougher, more porous device (like a teabag) holding associations of coated cells or globular capsules, and in that case the permeability requirement is conveniently a function of the encapsulation rather than of the fibre which can be stronger. Preferably the fibre has an active end, and an inactive end by means of which the implant may later be retrieved, Fig 5 shows at 500 such a fibre. The inactive end 502 includes an eyelet 501 and the active end is heat-sealed 504. The permeable portion of the fibre includes globular capsules 503 holding active cells (see Figs 3 or 4).

Implants may be distributed for use within a container holding a conventional liquid life-support media as is well known in the art. A kit of materials for surgical implantation of an implant may also be distributed in order to facilitate a sterile, slow virus-free operation. A minimal kit of materials might include a blade to guide a cannula into the ventricle, a cannula, and a container holding implants. A more comprehensive kit would also include drapes, skin preparation materials, scalpels, haemostats, a drill for obtaining surgical access through the cranium to the central nervous system, a blade guide, sutures, and dressings.

### EXAMPLE 3

Selection of choroid plexus cells capable of expressing a given balance of trophic factors, to use the term in a broad sense, is preferably done by selecting a particular species of mammal, and age of mammal from which the cells are to be harvested so that the cells of its choroid plexus already function as required. The age may be anywhere from perhaps mid-gestation or before, when a choroid plexus is identifiable, to somewhere in postnatal life. The output of the choroid plexus - in terms of trophic factors - changes during development of the brain through gestation and for perhaps a year afterwards. Myelination, for example, continues to proceed well after birth. Accordingly, modification of harvested cells in order to manipulate their properties as by restriction of the environment or by introduction of genetic material (DNA) is not expected. However, there may be instances when such steps are indicated. (Restriction, such as measures to adapt the cells to function within a relatively low pO2, is already provided for in this invention because a fetus has a lower pO2 in general). This invention may also be applied to cells taken from a human source. It may be possible to construct genetically modified and coated/protected choroid plexus cells for use in an xenotransplant designed for the purpose of compensating for a disease wherein inborn errors of metabolism affect the CNS wherein the implanted cells metabolise and thereby consume undesirable compounds, or compensate with products for other cells in the brain that fail to secrete desirable compounds, on the brain side of the "blood-brain barrier". This may be useful in lysosomal storage diseases or for other genetically based defects such as aspartoacyclase deficiency

### EXAMPLE 4

This example describes the use of a vascularised mechanical construction at least partially simulating the architecture of a choroid plexus; as a two-compartment form of "artificial organ". It may be located elsewhere in the body at a convenient, preferably subcutaneous or intraperitoneal site and surgically anastomosed between an artery and a vein. A tube, like a shunt as used for hydrocephalus, is connected between the artificial organ and a ventricle or the like, to carry the cerebrospinal fluid-like output from the device into the central nervous system. The usefulness of this approach is in part based on the possibility that a relatively large volume of choroid plexus materials, well vascularised, may be required to supply adequate amounts of both CSF fluid and trophic material for some neurological diseases.

Given that in nature the choroid plexus overlies an array of capillaries evidently exuding fluid, it may be useful to construct an implantable module including an artificial semi-permeable filter element exposed on one side to a flow of blood at an effective pressure, having on the other side an accumulation of choroid plexus epithelial cells optionally attached by means of an artificial basement membrane including a laminin or the like to be washed with the transudate, and a conduit for the transudate plus growth factors leading to the ventricular system of the brain. Preferably a filter is included in the outflow so that cellular material is not swept into the ventricle. It may be possible to either construct, or to cause the cells used in the artificial organ to mimic the extensively folded nature of the actual choroid plexus. In practice, there may be some usefulness in minimising a possible release of angiogenic factors backward from the active cells into the draining blood if the rate of flow is small, and usefulness in providing protection against an excessive quantity of fluid passing from the organ into the ventricle. In a worst case the fluid might comprise blood. An active control device, also capable of receiving external commands from time to time, may be included in the artificial organ and a precedent in life for such control means is the known extensive innervation received both by the vasculature and by the secretory cells of the choroid plexus.

### COMMERCIAL BENEFITS or ADVANTAGES

The pharmaceutical composition of this invention, when used for administration to patients suffering from a neurological disease or a disease causing degeneration of the CNS or parts thereof, may slow down or halt the disease (as a palliative treatment). This represents considerable personal, social and economic benefits. We expect that use of choroid plexus cells may even reverse the disease process by providing restorative treatment or possibly stimulating new growth of neurones and/or their processes.

In some instances the invention may simply provide extra CSF; presumably indirectly. In others, it may provide factors that are no longer naturally present in sufficient quantity to maintain neurones against "factors causing atrophy" and in some cases these factors may be provided only by choroid plexus cells of fetal or neonatal origin.

It may be that future applications simply comprise a rejuvenation of a relatively aged brain; in which instances the use of "new choroid plexus" reverses (to some extent) subclinical ageing processes.

Injuries to the central nervous system may benefit from trophic factors (and possibly also carriers) that can be produced by cell preparations such as those comprising this invention, inserted into the CNS.

Finally, we wish to reiterate that the description of various examples as provided in this specification is merely illustrative, and is not in any way to be taken as limiting of this invention as set forth in the following claims.

## Claims

1. An implantable devise comprising living cells collected from the choroid plexus of a mammal, at least some of which cells are choroid epithelial cells, said implant secreting at least one product, having a beneficial effect on a neurological disease when transplanted into a recipient mammal suffering from said neurological disease **characterised in that** the living cells are placed within a biocompatible containment means, wherein the living cells are encapsulated within a biocompatible capsule, the wall of which is at least partially composed of a semi-permeable membrane adapted to admit metabolites for sustaining the cells, while blocking access by factors of the immune system of the recipient mammal, and allowing an effective amount of one or more secreted products to exit from the implantable device.

2. An implantable device as claimed in claim 1, **characterised in that** the biocompatible capsule has an inner layer including an effective amount of a laminin; said laminin serving as a physical substrate for the living cells thereby providing orientation and support for the cells.

3. An implantable device as claimed in claim 2 **characterised in that** the biocompatible capsule comprises:
(a) a globular containment means holding living cells; or
(b) a tubular containment means holding living cells, the implantable device being adapted to be placed within a ventricle of the brain of the recipient mammal, so that the products secreted from the implantable device may access at least some regions of the central nervous system.

4. An implantable device as claimed in claim 1 **characterised in that** the living cells are taken from the choroid plexus of a fetal or neonatal mammal.

5. An implantable device as claimed in claim 4 **characterised in that** at least some living cells have undergone subsequent modification in order to increase the production of at least one product having a beneficial effect on a neurological disease.

6. An implantable device as claimed in claim 1, **characterised in that** the neurological disease is selected from the group consisting of Alzheimer's disease, Parkinson's disease, Huntington's disease, stroke, multiple sclerosis, aging, vascular disease, trauma or injury and damage to the nervous system.

7. An implantable device as claimed in claim 6. **characterised in that** the neurological disease is Huntington's disease or a stroke.

8. A use of mammalian choroid plexus cells in the manufacture of an implantable device as claimed in claim 1 for treating or preventing a neurological disorder in a mammal.

9. Use as claimed in claim 8 **characterised in that** the neurological disorder is selected from the group consisting of Alzheimer's disease, Parkinson's disease, Huntington's disease, stroke, multiple sclerosis, aging, vascular disease, trauma or injury and damage to the nervous system.

10. Use of isolated mammalian choroids plexus cells or neonatal choroid plexus cells in the implantable device of any one of claims 1 to 7.

11. An implantable device as claimed in claim 1, **characterised in that** said living cells are obtained from the choroid plexus of a neonatal porcine.

12. A use as claimed in claim 8 **characterised in that** said live cells are obtained from the choroid plexus of a neonatal porcine.

13. A vascularised device or artificial choroid plexus for use with the body of a mammal to be treated, **characterised in that** the vascularised device includes (a) means to connect a first, blood-bearing compartment of the device between an artery and a vein, (b) means to pass a fluid carrying means leading from a second, transudate-bearing compartment of the device to an implantable second end of the fluid carrying means, adapted to be implanted into a space within the brain containing cerebrospinal fluid, and (c) internal support means, comprising a permeable wall between the first compartment and the second compartment, said wall being adapted to support living cells collected from the choroid plexus of a mammal as claimed in claim 1, so that said living cells are exposed to transudate passing from the first compartment to the second compartment and so that said living cells express trophic factors into the transudate.

14. A container holding at least one implantable device as claimed in claim 1 within a container, **characterised in that** the container also includes a liquid media adapted to maintain the at least one implantable device in a living condition for a time.

15. A kit of materials, for surgical implantation of an implantable device as claimed in claim 1 in the central nervous system of a recipient mammal, **characterised in that** the kit of materials includes means for providing a sterile site, means for obtaining surgical access through the cranium to the central nervous sytem, means for haemostasis, means for placing at least one implantable device in an intended position, a container holding implantable devices as claimed in claim 14, means for closing off the surgical access site, and means for dressing the surgical access site, thereby to minimise the risk of introducing an infection during the operative procedure.

16. A kit of materials, for surgical implantation of an implantable device as claimed in claim 1, **characterised in that** the kit of materials includes means for placing at least one implantable device in an intended position and a container holding implantable devices as claimed in claim 14.

## Patentansprüche

1. Implantierbare Vorrichtung, welche dem Plexus Choroideus eines Säugetiers entnommene lebende Zellen umfasst, wobei mindestens einige der Zellen Choroid-Epithelzellen sind, wobei das Implantat mindestens ein Produkt absondert, das eine vorteilhafte Wirkung auf eine neurologische Erkrankung aufweist, wenn es in ein Empfängersäugertier transplantiert wird, das unter der neurologischen Erkrankung leidet, **dadurch gekennzeichnet, dass** die lebenden Zellen in ein biokompatibles Sicherheitsbehältermittel gesetzt sind, wobei die lebenden Zellen in einer biokompatiblen Kapsel eingeschlossen sind, deren Wand zumindest teilweise aus einer semipermeablen Membran besteht, die dafür ausgelegt ist, Metaboliten zum Versorgen der Zellen einzulassen, während sie durch Faktoren des Immunsystems des Empfängersäugetiers Zugang sperrt und eine wirksame Menge eines oder mehrerer abgesonderter Produkte aus der implantierbaren Vorrichtung austreten lässt.

2. Implantierbare Vorrichtung nach Anspruch 1, **dadurch gekennzeichnet, dass** die biokompatible Kapsel eine Innenschicht aufweist, die eine wirksame Menge eines Laminins umfasst, wobei das Laminin als physikalisches Substrat für die lebenden Zellen dient, wodurch für die Zellen Ausrichtung und Lagerung vorgesehen wird.

3. Implantierbare Vorrichtung nach Anspruch 2, **dadurch gekennzeichnet**, das die biokompatible Kapsel umfasst:
(a) ein kugelförmiges Sicherheitsbehältermittel, das lebende Zellen fasst; oder
(b) ein rohrförmiges Sicherheitsbehältermittel, das lebende Zellen fasst, wobei die implantierbare Vorrichtung dafür ausgelegt ist, in ein Himventrikel des Empfängersäugetiers gesetzt zu werden, so dass die aus der implantierbaren Vorrichtung abgesonderten Produkte zumindest in einige Bereiche des zentralen Nervensystems gelangen können.

4. Implantierbare Vorrichtung nach Anspruch 1, **dadurch gekennzeichnet, dass** die lebenden Zellen dem Plexus Choroideus eines fötalen oder neonatalen Säugetiers entnommen sind.

5. Implantierbare Vorrichtung nach Anspruch 4, **dadurch gekennzeichnet, dass** mindestens einige lebende Zellen eine anschließende Modifikation unterlaufen haben, um die Produktion mindestens eines Produkts mit einer vorteilhaften Wirkung auf eine neurologische Erkrankung zu steigern.

6. Implantierbare Vorrichtung nach Anspruch 1, **dadurch gekennzeichnet, dass** die neurologische Erkrankung gewählt ist aus der Gruppe bestehend aus Alzheimer-Krankheit, Parkinson-Krankheit, Huntington-Krankheit, Schlaganfall, Multiple Sklerose, Alterung, Gefäßerkrankung, Trauma oder Verletzung und Schädigung des Nervensystems.

7. Implantierbare Vorrichtung nach Anspruch 6, **dadurch gekennzeichnet, dass** die neurologische Erkrankung Huntington-Krankheit oder ein Schlaganfall ist.

8. Verwendung von Zellen des Plexus Choroideus von Säugetieren bei der Herstellung einer implantierbaren Vorrichtung nach Anspruch 1 zum Behandeln oder Verhindern einer neurologischen Krankheit in einem Säugetier.

9. Verwendung nach Anspruch 8, **dadurch gekennzeichnet, dass** die neurologische Krankheit gewählt ist aus der Gruppe bestehend aus Alzheimer-Krankheit, Parkinson-Krankheit, Huntington-Krankheit, Schlaganfall, Multiple Sklerose, Alterung, Gefäßerkrankung, Trauma oder Verletzung und Schädigung des Nervensystems.

10. Verwendung isolierter Zellen des Plexus Choroideus von Säugetieren oder neonataler Zellen des Plexus Choroideus in der implantierbaren Vorrichtung nach einem der Ansprüche 1 bis 7.

11. Implantierbare Vorrichtung nach Anspruch 1, **dadurch gekennzeichnet, dass** die lebenden Zellen aus dem Plexus Choroideus eines neonatalen Schweins gewonnen sind.

12. Implantierbare Vorrichtung nach Anspruch 8, **dadurch gekennzeichnet, dass** die lebenden Zellen aus dem Plexus Choroideus eines neonatalen Schweins gewonnen sind.

13. Vaskularisierte Vorrichtung oder künstlicher Plexus Choroideus zur Verwendung mit dem Körper eines zu behandelnden Säugetiers, **dadurch gekennzeichnet, dass** die vaskularisierte Vorrichtung umfasst: (a) Mittel zum Anschließen eines ersten, Blut enthaltenden Kompartiments der Vorrichtung zwischen einer Arterie und einer Vene, (b) Mittel zum Leiten eines Fluid befördernden Mittels, das von einem zweiten, Transudat enthaltenden Kompartiment der Vorrichtung zu einem implantierbaren zweiten Ende des Fluid befördernden Mittels führt, wobei es dafür ausgelegt ist, in einen Raum in dem Gehirn, der Rückenmarksflüssigkeit enthält, implantiert zu werden, und (c) interne Lagermittel, welche eine permeable Wand zwischen dem ersten Kompartiment und dem zweiten Kompartiment umfassen, wobei die Wand dafür ausgelegt ist, dem Plexus Choroideus eines Säugetiers entnommene lebende Zellen nach Anspruch 1 zu lagern, so dass die lebenden Zellen dem von dem ersten Kompartiment zu dem zweiten Kompartiment strömenden Transudat ausgesetzt sind und so dass die lebenden Zellen trophische Faktoren in das Transudat exprimieren.

14. Behälter, der mindestens eine implantierbare Vorrichtung nach Anspruch 1 in einem Behälter hält, **dadurch gekennzeichnet, dass** der Behälter auch ein flüssiges Medium umfasst, das so ausgelegt ist, dass es die mindestens eine implantierbare Vorrichtung für gewisse Zeit in einem lebenden Zustand hält.

15. Materialausrüstung für die chirurgische Implantation einer implantierbaren Vorrichtung nach Anspruch 1 in das zentrale Nervensystem eines Empfängersäugetiers, **dadurch gekennzeichnet, dass** die Materialausrüstung umfasst: Mittel zum Vorsehen einer sterilen Stelle, Mittel zum Erhalten von chirurgischem Zugriff durch den Schädel zu dem zentralen Nervensystem, Mittel für Blutstillung, Mittel zum Platzieren mindestens einer implantierbaren Vorrichtung in einer gewünschten Position, einen Behälter, der implantierbare Vorrichtungen nach Anspruch 14 fasst, Mittel zum Verschließen der chirurgischen Zugriffsstelle und Mittel zum Verbinden der chirurgischen Zugriffsstelle, wodurch das Risiko einer Infizierung während des operativen Vorgehens minimiert wird.

16. Materialausrüstung für die chirurgische Implantation einer implantierbaren Vorrichtung nach Anspruch 1, **dadurch gekennzeichnet, dass** die Materialausrüstung Mittel zum Platzieren mindestens einer implantierbaren Vorrichtung in einer erwünschten Position und einen Behälter, der implantierbare Vorrichtungen nach Anspruch 14 fasst, umfasst.

## Revendications

1. Dispositif implantable comprenant des cellules vivantes collectées à partir du plexus choroïde d'un mammifère, dont au moins certaines cellules sont des cellules épithéliales choroïdiennes, ledit implant sécrétant au moins un produit, ayant un effet bénéfique sur une maladie neurologique lorsqu'il est transplanté dans un mammifère receveur souffrant de ladite maladie neurologique **caractérisé en ce que** les cellules vivantes sont placées dans un moyen de confinement biocompatible, dans lequel les cellules vivantes sont encapsulées dans une capsule biocompatible, dont la paroi est au moins partiellement composée d'une membrane semi-perméable adaptée pour permettre aux métabolites de nourrir les cellules, tout en bloquant l'accès à des facteurs du système immunitaire du mammifère receveur, et en permettant à une quantité efficace d'un ou plusieurs produits sécrétés de sortir du dispositif implantable.

2. Dispositif implantable tel que revendiqué dans la revendication 1, **caractérisé en ce que** la capsule biocompatible a une couche interne incluant une quantité efficace d'une laminine ; ladite laminine servant de substrat physique pour les cellules vivantes fournissant ainsi une orientation et un support pour les cellules.

3. Dispositif implantable tel que revendiqué à la revendication 2, **caractérisé en ce que** la capsule biocompatible comprend :
(a) un moyen de confinement globulaire maintenant les cellules vivantes ; ou
(b) un moyen de confinement tubulaire maintenant les cellules vivantes, le dispositif implantable étant adapté pour être placé dans un ventricule cérébral de l'animal receveur, de sorte que les produits sécrétés à partir du dispositif implantable puissent accéder à au moins certaines régions du système nerveux central.

4. Dispositif implantable tel que revendiqué à la revendication 1, **caractérisé en ce que** les cellules vivantes sont prélevées du plexus choroïde d'un mammifère foetal ou néonatal.

5. Dispositif implantable tel que revendiqué à la revendication 4, **caractérisé en ce qu'**au moins certaines cellules vivantes ont subi une modification ultérieure afin d'accroître la production d'au moins un produit ayant un effet bénéfique sur une maladie neurologique.

6. Dispositif implantable tel que revendiqué à la revendication 1, **caractérisé en ce que** la maladie neurologique est choisie dans le groupe constitué de la maladie d'Alzheimer, la maladie de Parkinson, la maladie de Huntington, un accident vasculaire cérébral, la sclérose en plaques, le vieillissement, une maladie vasculaire, un traumatisme ou une blessure et une dégradation du système nerveux.

7. Dispositif implantable tel que revendiqué à la revendication 6, **caractérisé en ce que** la maladie neurologique est la maladie de Huntington ou un accident vasculaire cérébral.

8. Utilisation de cellules de plexus choroïde d'un mammifère dans la fabrication d'un dispositif implantable tel que revendiqué à la revendication 1 pour le traitement ou la prévention d'un trouble neurologique chez un mammifère.

9. Utilisation telle que revendiquée à la revendication 8, **caractérisée en ce que** le trouble neurologique est choisi dans le groupe constitué de la maladie d'Alzheimer, la maladie de Parkinson, la maladie de Huntington, un accident vasculaire cérébral, la sclérose en plaques, le vieillissement, une maladie vasculaire, un traumatisme ou une blessure et une dégradation du système nerveux.

10. Utilisation de cellules isolées de plexus choroïde d'un mammifère ou de cellules de plexus choroïde néonatal dans un dispositif implantable de l'une quelconque des revendications 1 à 7.

11. Dispositif implantable tel que revendiqué à la revendication 1, **caractérisé en ce que** lesdites cellules vivantes sont obtenues à partir du plexus choroïde d'un porcin néonatal.

12. Utilisation telle que revendiquée à la revendication 8, **caractérisée en ce que** lesdites cellules vivantes sont obtenues à partir du plexus choroïde d'un porcin néonatal.

13. Dispositif vascularisé ou plexus choroïde artificiel utilisable dans le corps d'un mammifère à traiter, **caractérisé en ce que** le dispositif vascularisé comprend (a) un moyen pour connecter un premier compartiment du dispositif véhiculant du sang entre une artère et une veine, (b) un moyen pour faire passer un moyen transportant un fluide conduisant d'un second compartiment véhiculant un transudat du dispositif vers une seconde extrémité implantable du moyen portant le fluide, adapté pour être implanté dans un espace à l'intérieur du cerveau contenant du liquide céphalo-rachidien, et (c) un moyen de support interne, comprenant une paroi perméable entre le premier compartiment et le second compartiment, ladite paroi étant adaptée pour supporter des cellules vivantes récoltées à partir du plexus choroïde d'un mammifère tel que revendiqué à la revendication 1, de sorte que lesdites cellules vivantes soient exposées au transudat passant du premier compartiment vers le second compartiment et de sorte que lesdites cellules vivantes expriment des facteurs trophiques dans le transudat.

14. Récipient contenant au moins un dispositif implantable tel que revendiqué à la revendication 1 dans un récipient, **caractérisé en ce que** le récipient comprend également un milieu liquide adapté pour maintenir au moins un dispositif implantable dans un état vivant pendant un certain temps.

15. Kit de matériels, pour implantation chirurgicale d'un dispositif implantable tel que revendiqué à la revendication 1 dans le système nerveux central d'un mammifère receveur, **caractérisé en ce que** le kit de matériaux comprend un moyen pour fournir un site stérile, un moyen pour obtenir un accès chirurgical par le crâne au système nerveux central, un moyen pour hémostase, un moyen pour placer au moins un dispositif implantable dans une position voulue, un récipient contenant des dispositifs implantables tel que revendiqué à la revendication 14, un moyen pour refermer le site d'accès chirurgical, et un moyen pour panser le site d'accès chirurgical, minimisant ainsi le risque d'introduire une infection durant le processus opératoire.

16. Kit de matériels pour implantation chirurgicale d'un dispositif implantable tel que revendiqué à la revendication 1, **caractérisé en ce que** le kit de matériels comprend un moyen pour placer au moins un dispositif implantable en une position voulue et un récipient contenant des dispositifs implantables tel que revendiqué à la revendication 14.
